# EUROPEAN PATENT APPLICATION

(11) **EP 2 638 882 A1**
(43) Date of publication of application: **18.09.2013**
(21) Application number: 11839759.5
(22) Date of filing: 16.08.2011
(51) Int. Cl.: A61F 2/82

(54) **BIFURCATION BLOOD VESSEL STENT**

(30) Priority: 12.11.2010 CN 201010543016
(71) Applicant: Shanghai MicroPort Medical (Group) Co., Ltd., Shanghai 201203 (CN)
(72) Inventor: CAI, Xu, Shanghai 201203 (CN); ZHANG, Dadong, Shanghai 201203 (CN); WANG, Changchun, Shanghai 201203 (CN); TANG, Zhirong, Shanghai 201203 (CN); LUO, Qiyi, Shanghai 201203 (CN); CHANG, Zhaohua, Shanghai 201203 (CN)
(74) Representative: Dantz, Jan Henning
(86) International application number: PCT/CN2011/078445
(87) International publication number: WO 2012/062144

(57) **Abstract**

A stent for a bifurcated vessel comprises a stent body (1) having two open ends (101,102), wherein at least one of the open ends (101,102) of the stent body is a wedge structure, a second opaque marker (6) is provided on the two ends of the slant long axis of the wedge structure, the stent body (1) is provided with two first opaque markers (5), and the connecting line between the two first opaque markers (5) is parallel to the slant of the wedge structure. Since the connecting line between the two first opaque markers (5) on the stent for a bifurcated vessel is parallel to the slant of the wedge structure, when the two first opaque markers (5) overlap with each other, the slant of the wedge structure of the stent for a bifurcated vessel is precisely positioned during implantation so that the slant of the wedge structure of the stent for a bifurcated vessel fits the slant at the blood vessel bifurcation site.

## Description

### Technical Field

The present invention is related to a medical device, particularly a stent for a bifurcated vessel.

### Background

An intravascular stent is usually a drug eluting stent used for the treatment of vascular stenosis. By providing support to a pathological vessel, an intravascular stent implanted into a human body can facilitate the restoration of the pathological vessel to a normal state. Meanwhile, medicament on the stent is released to the vascular wall that is in contact with the stent so as to suppress the growth of cells of the vascular wall, and thereby to lower down the incidences of recurrent vascular stenosis.

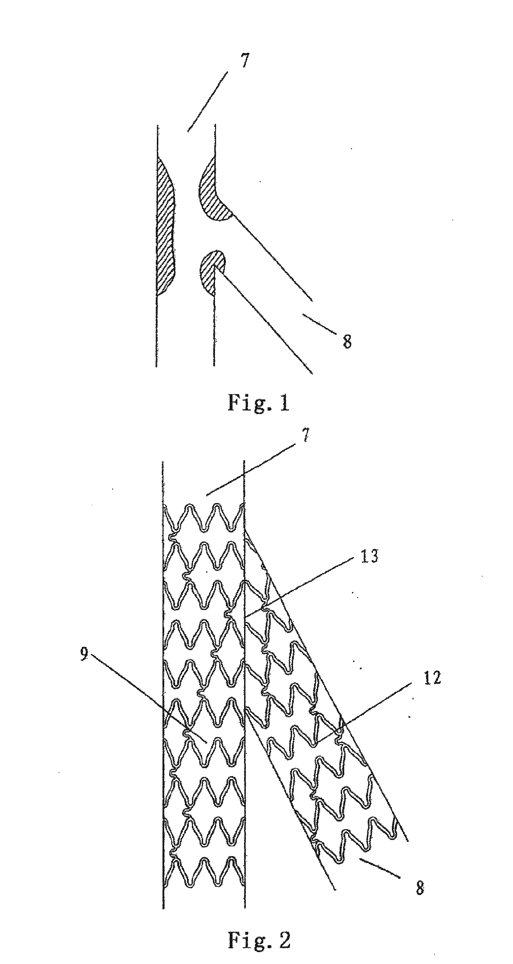

In clinical practice, vascular stenosis in many patients occurs not only at one site but at multiple sites of the blood vessel. Bifurcation lesion as indicated by the shadow areas in Fig. 1 is a common multiple-site artery stenosis wherein the vascular lesion site is located at the bifurcation between main vessel 7 and branch vessel 8. At present, a stent for a bifurcated vessel with a wedge-shaped end is typically employed for the treatment of bifurcation lesions. As shown in Fig. 2, the main-vessel stent is denoted by the reference sign 9, the stent for a bifurcated vessel is denoted by the reference sign 12, and the wedge-shaped structure on the stent for a bifurcated vessel is denoted by the reference sign 13. When the stent for a bifurcated vessel 12 is implanted into the human body, the wedge-shaped structure 13 on the stent for a bifurcated vessel 12 is so fitted to the main vessel stent 9 that the pathological vessel at the bifurcation site is completely and sufficiently covered, and the stents will not overlap at the bifurcation site. Therefore the occurrence of thrombus due to excessive metal being implanted at the bifurcation site can be prevented.

After investigating the prior art of the field, the inventor has found that although the existing stent for a bifurcated vessel with a wedge structure is capable of providing excellent treatment to the bifurcation lesion, the slant of the wedge structure of the stent for a bifurcated vessel cannot be positioned properly. In other words, it is impossible to guarantee a proper fit between the slant of the wedge structure of the stent for a bifurcated vessel and the main-vessel stent. Thereby the slant section of the implanted stent may deviate from the main vessel, which is detrimental to therapeutic effect.

### The Content of the Invention

Based on the investigation, a stent for a bifurcated vessel is provided in embodiments of the present application, wherein a first opaque point and a second opaque point are disposed on the stent body in order to solve the problem associated with the existing stent for a bifurcated vessel that it cannot be precisely positioned during implantation.

To achieve the above object, the present application provides technical solutions as follows:
A stent for a bifurcated vessel comprises a stent body having two open ends, wherein at least one of the open ends of the stent body is a wedge structure, a second opaque marker is provided on the two ends of the long axis of the slant of the wedge structure, the stent body is provided with two first opaque markers, and the connecting line between the two first opaque markers is parallel to the slant of the wedge structure.

Preferably, said two first opaque markers are located at the other open end of the stent body.

Preferably, the distance between said two first opaque markers equals to the diameter of said stent body.

Preferably, when the number of said second opaque marker is one, said second opaque marker is located at any one end of the long axis of the slant of the wedge structure; when the number of said second opaque marker is two, said second opaque markers are located at both ends of the long axis of the slant of the wedge structure.

Preferably, protruded tooth-like structures are provided around the slant of said wedge structure.

Preferably, the axial length of said wedge structure is 1-15mm.

Preferably, the angle between the slant of said wedge structure and the axial direction of said stent body is 5-85 degree.

Preferably, the diameter of said stent for a bifurcated vessel is 2.25mm-10.0mm.

Preferably, said stent for a bifurcated vessel is a coronary artery bifurcation stent.

Preferably, the diameter of said coronary artery bifurcation stent is 2.25mm-5.0mm.

Preferably, said stent body is made of a material with excellent biocompatibility and mechanical properties selected from stainless steel, cobalt-chromium alloy, nickel-based alloy, degradable magnesium alloy or polymeric materials.

As can be seen from the above technical solutions, the examples of the present application provide a stent for a bifurcated vessel comprising a stent body having two open ends, wherein at least one of the open ends of the stent body is a wedge structure, the stent body is provided with two first opaque markers, and the connecting line between the two first opaque markers is parallel to the slant of the wedge structure, at least one second opaque marker is provided on the two ends of the long axis of the slant of the wedge structure. During implanting the stent for a bifurcated vessel into a bifurcation vascular, the operator firstly delivers the wedge structure of the stent for a bifurcated vessel precisely to the bifurcation site of the blood vessel by observing the second opaque marker under X-ray, and then the operator can adjust the stent for a bifurcated vessel by observing the two first opaque markers under X-ray to overlap the two first opaque markers. Since the connecting line between the two first opaque markers is parallel to the slant of the wedge structure, the operator can precisely position the wedge structure of the stent for a bifurcated vessel by observing the first opaque markers so that the slant of the wedge structure of the stent for a bifurcated vessel fits the slant at the blood vessel bifurcation site.

Therefore, the stent for a bifurcated vessel provided herein can avoid the problem associated with the existing stent for a bifurcated vessel that the wedge structure deviates from the main vessel during implantation.

### Brief Description of Drawings

To better illustrate the examples in the present invention or the technical solutions in the prior art, the figures needed in the description of the Examples or the prior art will be briefly described as follows. It is obvious that the figures below only represent a few of the examples recorded in the application and a skilled in the art can obtain other figures according to what have been shown here without paying any inventive effort.
Fig.1 is a schematic diagram showing lesion sites in a common bifurcated lesion;
Fig. 2 is a schematic diagram showing an operation of a wedge structure of an existing stent for a bifurcated vessel;
Fig. 3 is a schematic structural diagram showing the cross-section of a stent for a bifurcated vessel of an embodiment of the present application;
Fig. 4 schematically shows the three-dimensional structure of the stent shown in Fig. 3;
Fig. 5 is a schematic structural diagram showing the cross-section of another stent for a bifurcated vessel of the embodiment of the present application;
Fig. 6 is a schematic structural diagram showing the cross-section of a third stent for a bifurcated vessel of the embodiment of the present application;
Fig. 7 is a structural diagram showing a stent for a bifurcated vessel of the embodiment of the present application being fixed by a balloon dilation catheter;
Fig. 8 is a schematic diagram showing an operation of a stent for a bifurcated vessel of the embodiment of the present application.

### Detail Description of the Invention

For a skilled in the art to better under the technical solutions in the application, technical solutions in the application will be described thoroughly hereinafter in more detail with reference to the figures herein. It is clear that the examples described herein represent only a few examples instead of all of the examples of the application. All the other examples obtained based on the examples of the present application by one ordinary skilled in the art without paying any inventive effort are deemed to fall into the scope of protection of the present application.

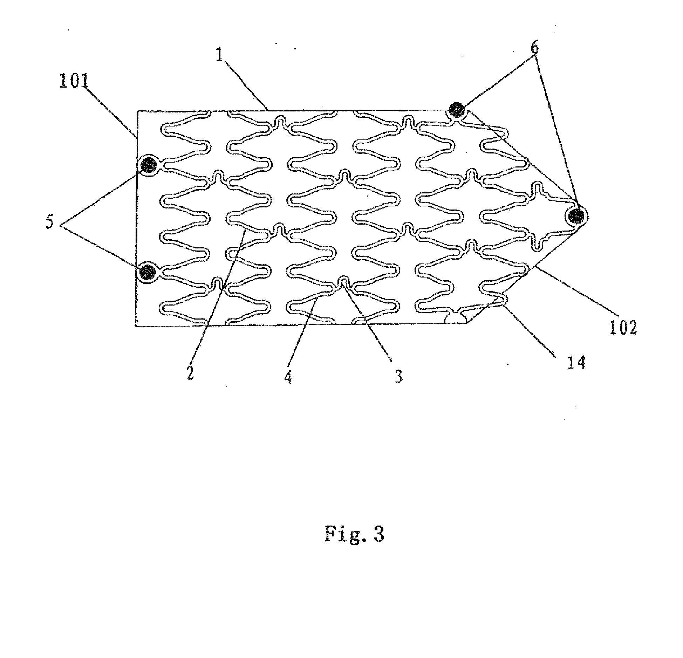

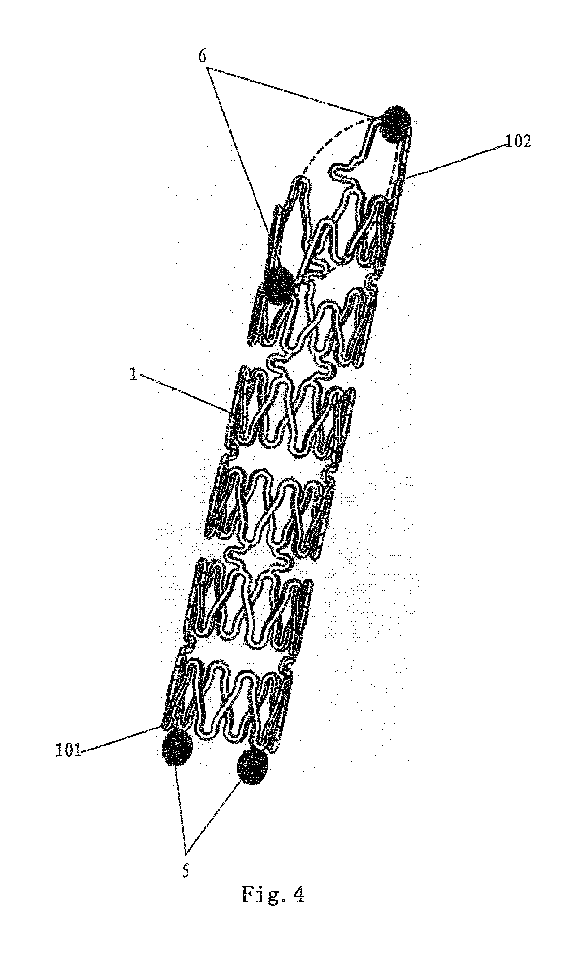

Fig. 3 is the first schematic structural diagram showing the cross-section of a stent for a bifurcated vessel of the embodiment of the present application. Fig. 4 is the second schematic structural diagram showing the cross-section of a stent for a bifurcated vessel of the embodiment of the present application.

As shown in Figs. 3 and 4, the stent for a bifurcated vessel comprises a stent body 1 having two open ends, namely a first open end 101 and a second open end 102, in which the second open end 102 is a wedge-shaped structure. However in the embodiments of the present application, depending on the angles of the blood vessel bifurcation of the patient, the angle between the slant of the wedge structure and the axial direction of the stent body 1 is preferably 45 degree, and the axial length of the wedge structure is within the range of 1-15mm.

The stent body 1 is composed of multiple sets of mental loop 2 and connecting stems 3. Each set of mental loop 2 is formed by connecting multiple wavelike rods 4 together and the connecting stems 3 is positioned between the adjacent mental loops 2 to connect the adjacent mental loops 2. The stent for a bifurcated vessel is suitable for various parts of a human body, such as cerebral artery bifurcation stent, femoral artery bifurcation stent, coronary artery bifurcation stent and the like. In the embodiment of the present application, the diameter of the stent for a bifurcated vessel is preferably 2.25mm-10.0mm. Further, the stent for a bifurcated vessel is preferably a coronary artery bifurcation stent and the diameter of the coronary artery bifurcation stent is 2.25mm-5.0mm. In other embodiments of the present application, the stent body 1 can also be made by weaving metal filaments or formed by etching tubular materials. In addition, the stent body 1 is made of a material with excellent biocompatibility and mechanical properties, such as stainless steel, cobalt-chromium alloy, nickel-based alloy, degradable magnesium alloy or polymeric materials etc.

As shown in Figs. 3 and 4, two second opaque markers 6 are provided on the second open end 102 of the stent 1, and the second opaque markers are positioned on the opposite ends of the long axis of the slant of the wedge structure. Further, two first opaque markers 5 are provided on the stent 1, and the connecting line between the two first opaque markers 5 is parallel to the slant of the wedge structure. In the embodiments of the present application, the two first opaque markers 5 are preferably located at the first open end 101, and preferably, the distance between the two first opaque markers 5 equals to the diameter of the stent body 1.

In the embodiments of the present application, the first opaque marker 5 and the second opaque marker 6 can be opaque metal sheet embedded on the wavelike rod 4 of the stent, or opaque film coated on the wavelike rod 4 of the stent, or opaque filament winded on the wavelike rod 4 of the stent.

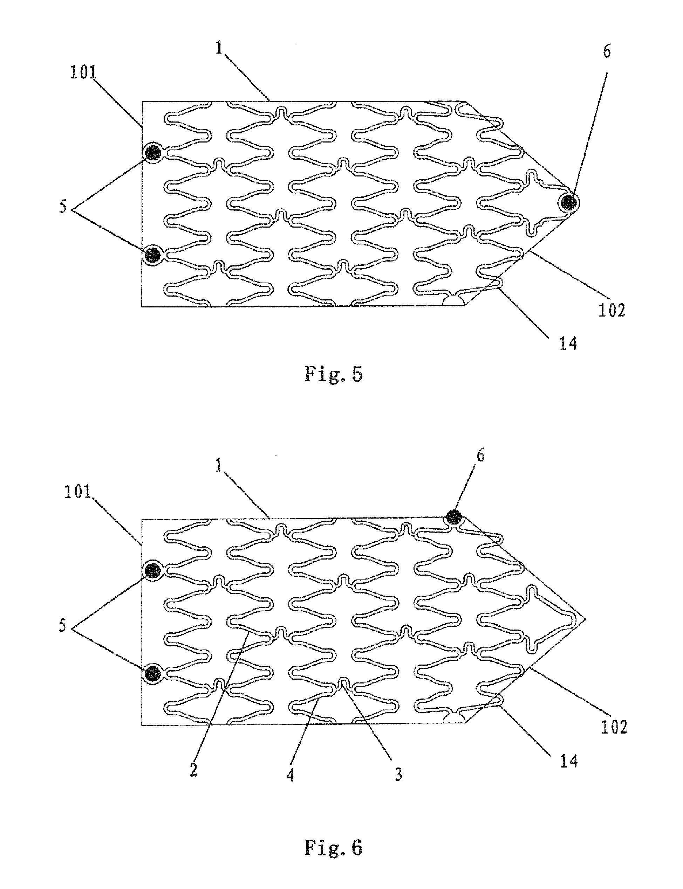

In other embodiments of the present application, the number of the second opaque marker 6 can be one and the second opaque marker 6 is located at any one end of the long axis of the slant of the wedge structure. As shown in Fig. 5, which is a schematic structural diagram of another stent for a bifurcated vessel of the embodiment of the present application, one second opaque marker 6 is provided and located at the bottom of the wedge structure (the side close to the top end of the second open end 102 is defined as top). Fig. 6, which is a schematic structural diagram of a third stent for a bifurcated vessel of the embodiment of the present application, also shows that one second opaque marker 6 is provided and located at the top of the wedge structure. In the embodiments of the present application, since the second opaque marker 6 is provided on the two ends of the long axis of the slant of the wedge structure, the development thereof under X-ray will indicate the position of the wedge structure of the stent for a bifurcated vessel, no matter one or two second opaque markers 6 being disposed on the stent.

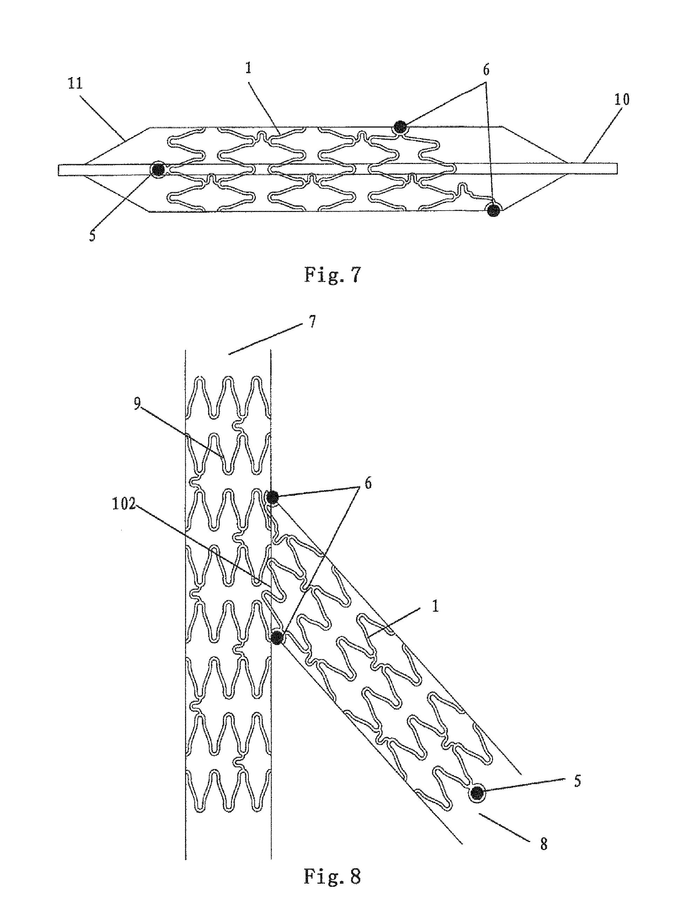

Fig. 8 is a schematic diagram showing an operation of a stent for a bifurcated vessel of the embodiment of the present application.

As shown in Fig. 8, vascular stenosis occurs in both main vessel 7 and branch vessel 8, a main-vessel stent is denoted by the reference sign 9. The implantation of the stent is performed in combination with the use of the balloon dilation catheter 10. As shown in Fig. 7, the stent for a bifurcated vessel is first compressed to be in tight contact with the balloon 11 of the balloon dilation catheter 10 before implantation; and then the balloon dilation catheter 10 is guided to the branch vessel 8 through the main vessel 7; the wedge structure of the stent body 1 is delivered precisely to the vascular bifurcation site by moving back and forth the balloon dilation catheter 10 based on the second opaque marker 6 under X-ray; and the stent for a bifurcated vessel is adjusted so that the wedge structure of the stent body 1 and the main-vessel stent 9 is jointed together based on the first opaque marker 5 under X-ray. The jointing procedure is described as follows: the operator observes whether the two first opaque points 5 overlap under X-ray. If the two first opaque points 5 do not overlap, the operator turns the balloon dilation catheter 10 so that the two first opaque points 5 will overlap. Due to the fact that the connecting line between the two first opaque points 5 is parallel to the slant of the wedge structure, the projection of the slant of the wedge structure is shown as a line in the perspective of the operator when the two first opaque points 5 overlap. This means that the joint between the slant of the wedge structure and the main-vessel stent 9 is accomplished. When the jointing procedure is finished, the stent for a bifurcated vessel is then released and dilated to completely cover the vascular lesion site as shown in Fig. 8. Then the implantation process is completed.

In addition, as shown in Fig. 3, which is a schematic structural diagram of the stent for a bifurcated vessel of the embodiments of the present application, protruded tooth-like structures 14 are provided on the wedge structure of the second open end 102. The tooth-like structures 14 are arranged around the slant of the wedge structure and the extending direction thereof can be parallel to that of the stent body 1 or can extend outward. Therefore, when the wedge structure and the main-vessel stent 9 are jointed together after implantation, the tooth-like structures 14 on the wedge structure can be inserted into the main-vessel stent 9 or cover the opening of the main-vessel stent 9, so that the stent for a bifurcated vessel can more tightly contact the main-vessel stent 9.

As can be seen from the above technical solutions, at least one open end of the stent body of the stent for a bifurcated vessel provided in the embodiments of the present application is a wedge structure. Two first opaque markers are provided on the stent body, and the connecting line between the two first opaque markers is parallel to the slant of the wedge structure. At least one second opaque marker is provided on the two ends of the long axis of the slant of the wedge structure. When implanting the stent for a bifurcated vessel into the branch vessel, the operator firstly can precisely deliver the wedge structure of the stent for a bifurcated vessel to the bifurcation site of the blood vessel by observing the second opaque marker under X-ray. Then the operator adjusts the stent for a bifurcated vessel by observing the two first opaque markers under X-ray to overlap the two first opaque markers. Since the connecting line between the two first opaque markers is parallel to the slant of the wedge structure, the operator can precisely position the wedge structure of the stent for a bifurcated vessel by observing the first opaque markers so that the slant of the wedge structure of the stent for a bifurcated vessel matches the slant at the blood vessel bifurcation site.

Therefore, the stent for a bifurcated vessel provided herein can avoid the problem associated with the existing stent for a bifurcated vessel that the wedge structure deviates from the main vessel during implantation.

The above-mentioned examples are only preferred embodiments of the present application which are used to help understand or practice the present application. It is obvious for those skilled in the art that various modifications to these embodiments can be made and general principles defined herein can be embodied in other examples without departing from the spirit or scope of the present application. Therefore, the present application will not be limited to these examples demonstrated herein but claims the broadest scope that is in line with the principles and novel features disclosed herein.

## Claims

1. A stent for a bifurcated vessel comprising a stent body having two open ends, wherein at least one of the open ends of the stent body is a wedge structure, a second opaque marker is provided on the two ends of the long axis of the slant of the wedge structure, the stent body is provided with two first opaque markers, and the connecting line between the two first opaque markers is parallel to the slant of the wedge structure.

2. The stent for a bifurcated vessel according to claim 1, wherein said two first opaque markers are located at the other open end of the stent body.

3. The stent for a bifurcated vessel according to claim 2, wherein the distance between said two first opaque markers equals to the diameter of said stent body.

4. The stent for a bifurcated vessel according to claim 1, wherein the number of said second opaque marker is one or two, and when the number of said second opaque marker is one, said second opaque marker is located at any one end of the long axis of the slant of the wedge structure, and when the number of said second opaque marker is two, said second opaque markers are located at both ends of the long axis of the slant of the wedge structure.

5. The stent for a bifurcated vessel according to claim 1, wherein protruded tooth-like structures are provided around the slant of said wedge structure.

6. The stent for a bifurcated vessel according to claim 1, wherein the axial length of said wedge structure is 1-15mm.

7. The stent for a bifurcated vessel according to claim 6, wherein the angle between the slant of said wedge structure and the axial direction of said stent body is 5-85 degree.

8. The stent for a bifurcated vessel according to claim 1, wherein the diameter of said stent for a bifurcated vessel is 2.25mm-10.0mm.

9. The stent for a bifurcated vessel according to claim 8, wherein said stent for a bifurcated vessel is a coronary artery bifurcation stent.

10. The stent for a bifurcated vessel according to claim 9, wherein the diameter of said coronary artery bifurcation stent is 2.25mm-5.0mm.

11. The stent for a bifurcated vessel according to claim 1, wherein said stent body is made of a material with excellent biocompatibility and mechanical properties selected from stainless steel, cobalt-chromium alloy, nickel-based alloy, degradable magnesium alloy or polymeric materials.
